(19) Europäisches Patentamt
European Patent Office
Office européen des brevets

(11) Veröffentlichungsnummer: **0 126 183**
**A1**

(12) # EUROPÄISCHE PATENTANMELDUNG

(21) Anmeldenummer: **83112433.4**

(22) Anmeldetag: **10.12.83**

(51) Int. Cl.[3]: **C 07 D 233/92**, C 07 D 233/93, C 07 D 233/94, C 07 D 233/95, C 07 D 405/06, A 61 K 31/415

(30) Priorität: **23.12.82 DE 3247647**
**23.12.82 DE 3247646**

(43) Veröffentlichungstag der Anmeldung: **28.11.84 Patentblatt 84/48**

(84) Benannte Vertragsstaaten: **AT BE CH DE FR GB IT LI LU NL SE**

(71) Anmelder: **A. Nattermann & Cie. GmbH, Nattermannallee 1, D-5000 Köln 30 (DE)**

(72) Erfinder: **Dereu, Norbert, Dr. Dipl.-Chem., An der Holzhecke 11, D-5020 Frechen (DE)**
Erfinder: **Weiter, André, Dr. Dipl.-Chem., Reiherweg 11 a, D-5024 Pulheim (DE)**
Erfinder: **Ghyczy, Miklos, Dr. Dipl.-Chem., Am Serviesberg 12, D-5000 Köln 41 (DE)**

(74) Vertreter: **Sternagel, Hans-Günther, Dr. et al, Patentanwälte Dr. M. Hann Dr. H.-G. Sternagel Marburger Strasse 38, D-6300 Giessen (DE)**

(54) **Substituierte 5(4)-Nitroimidazole sowie Verfahren zu ihrer Herstellung.**

(57) Substituierte 5(4)-Nitroimidazole der Formel I

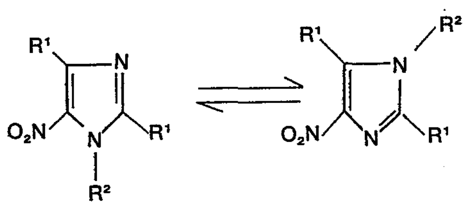

I

worin $R^1$ $CHR^3R^4$ oder Cycloalkyl, $R^2$ Wasserstoff, $C_{1-16}$-Alkyl oder –A–Y mit Y = H, $-XR^5$ oder Halogen bei A = Alkylen,
|
Y
X = O, S, $SO_2$, $SO_3$, $NR^5$ und $R^5$ = H oder $C_{1-8}$-Alkyl bedeuten, sowie Verfahren zu ihrer Herstellung.

Die neuen Verbindungen können als Pharmazeutika und Zwischenprodukte zur Herstellung von Arzneimitteln verwendet werden.

EP 0 126 183 A1

**PATENTANWÄLTE**
**DR. MICHAEL HANN**
**DR. H.-G. STERNAGEL**
**MARBURGER STRASSE 38**
**6300 GIESSEN 1**

(E-1663) St/Is

Anmelder: A. Nattermann & Cie GmbH
Nattermannallee 1, 5000 Köln 30

Titel: Substituierte 5(4)-Nitroimidazole sowie Verfahren zu ihrer Herstellung

## Beschreibung

Die Erfindung betrifft neue 1,2,4(5)-substituierte 5(4)-Nitroimidazole sowie Verfahren zu ihrer Herstellung. Die neuen Verbindungen eignen sich als Pharmazeutika, Pflanzenschutz- und Schädlingsbekämpfungsmittel, sowie als Zwischenprodukte zur Herstellung von Arzneimitteln.

1-Substituierte 5-Nitroimidazole sind vielfach beschrieben und werden als Arzneimittel, insbesondere als Chemotherapeutika, eingesetzt. Beispiele sind z.B. Metronidazol, Ornidazol und Ternidazol.
Bisher nicht beschrieben sind jedoch 1,2,4(5)-substituierte 5(4)-Nitroimidazole, die in der 2 und in der 4- oder 5-Stellung jeweils eine Gruppe tragen, die mit einem sekundären Kohlenstoffatom am Imidazolring gebunden ist.

Gegenstand der Erfindung sind neue 1,2,4(5)-substituierte 5(4)-Nitroimidazole der Formel I

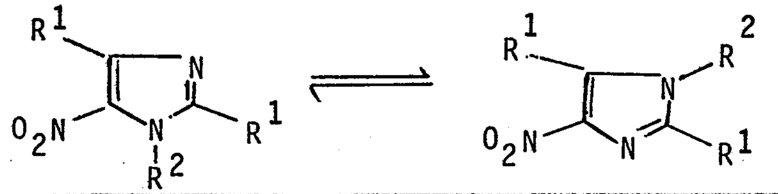

I

worin

$R^1$ die Gruppe $-CHR^3R^4$ oder eine Cycloalkylgruppe mit 3 - 7 Kohlenstoffatomen

$R^3$, $R^4$ einen Alkylrest mit 1 - 18, insbesondere 1 - 3 Kohlenstoffatomen

$R^2$ Wasserstoff, einen geradkettigen oder verzweigten, gesättigten oder ungesättigten Alkylrest mit 1 - 16 Kohlenstoffatomen, eine Epoxyalkylgruppe oder die Gruppe $-\underset{\underset{Y}{|}}{A}-Y$, in der

A entweder eine geradkettige oder verzweigte, monosubstituierte Alkylengruppe oder eine disubstituierte Alkylengruppe

Y Wasserstoff, $-XR^5$ oder Halogen

X O, S, $SO_2$, $SO_3$ oder $-NR^5$

$R^5$ Wasserstoff oder einen geradkettigen oder verzweigten Alkylrest mit 1 - 8, insbesondere 1 - 4 Kohlenstoffatomen

bedeuten.

Als Alkylengruppe A für die Monosubstitution kommen z.B. in Frage: Methylen, Ethylen, Trimethylen, Tetramethylen, 1-Methylethylen, 1-Methyltrimethylen, 1-Methyltetramethylen, 2-Methylpropylen, 2-Methyltetramethylen, 2-Methylpentamethylen. Für die Disubstitution wird in 2- und 3-Stellung substituiertes Propylen bevorzugt.

Als Beispiele für die Reste $R^2$ seien genannt Wasserstoff, Methyl, Ethyl, Isopropyl, Butyl, Isobutyl, 2-Hydroxyethyl, 3-Hydroxypropyl, 4-Hydroxybutyl, 2-Chlorethyl, 3-Chlorpropyl, 2,3-Dihydroxypropyl, 3-Chlor-2-hydroxypropyl, 2,3-Epoxy- propyl, 2-Hydroxypropyl, Hexadecyl, 2-Aminoethyl, 3-Aminopropyl, Dimethylaminoethyl, Dimethylaminopropyl, Diethylaminopropyl, Diisopropylaminoethyl,

2-Mercaptoethyl, 3-Mercaptopropyl, 2-Bromethyl, 3-Brompropyl, Methoxymethyl, 2-Methoxyethyl, 3-Methoxypropyl, Methylthiomethyl, 2-Methylthioethyl, 2-Methylthioethyl-S-dioxid, 3-Methylthiopropyl.

Erfindungsgemäße Verbindungen sind beispielsweise:
2,4(5)-Diisopropyl-5(4)-nitroimidazol,
2,4(5)-Di -sec-butyl-5(4)-nitroimidazol,
2,4(5)-Bis-(1-ethylpropyl)-5(4)-nitroimidazol,
2,4(5)-Bis-(1-methylbutyl)-5(4)-nitroimidazol,
2,4(5)-Bis-(1-methylhexyl)-5(4)-nitroimidazol,
2,4(5)-Bis-(1-methylhexadecyl)-5(4)-nitroimidazol,
2,4(5)-Dicyclopropyl-5(4)-nitroimidazol,
2,4(5)-Dicyclobutyl-5(4)-nitroimidazol,
2,4(5)-Dicyclopentyl-5(4)-nitroimidazol,
2,4(5)-Dicyclohexyl-5(4)-nitroimidazol,
2,4-Diisopropyl-1-methyl-5-nitroimidazol,
2,5-Diisopropyl-1-methyl-4-nitroimidazol,
2.4-Dicyclopropyl-1-methyl-5-nitroimidazol,
2,4-Dicyclohexyl-1-methyl-5-nitroimidazol,
2,4-Diisopropyl-1-ethyl-5-nitroimidazol,
2,4-Diisopropyl-1-propyl-5-nitroimidazol,
1-Butyl-2,4-diisopropyl-5-nitroimidazol,
1-Butyl-2,4-dicyclopentyl-5-nitroimidazol,
2,4-Diisopropyl-1-isopropyl-5-nitroimidazol,
2,4-Diisopropyl-1-(2-hydroxyethyl)-5-nitroimidazol,
2,4-Bis-(1-ethyl-propyl)-1-(2-hydroxyethyl)-5-nitroimidazol,
2,4-Dicyclopropyl-1-(2-hydroxyethyl)-5-nitroimidazol,
2,4-Dicyclohexyl-1-(2-hydroxyethyl)-5-nitroimidazol,
2,4-Diisopropyl-1-(2-hydroxypropyl)-5-nitroimidazol,
2,4-Bis-(1-methyl-propyl)-1-(2-hydroxypropyl)-5-nitroimidazol,
2,4-Dicyclopropyl-1-(2-hydroxpropyl)-5-nitroimidazol,
1-(3-Chlor-2-hydroxypropyl)-2,4-diisopropyl-5-nitroimidazol,
2,4-Bis-(1-ethyl-propyl)-1-(3-chlor-2-hydroxypropyl)-5-nitroimidazol,

1-(3-Chlor-2-hydroxypropyl)-2,4-diisopropyl-5-nitroimidazol,
1-(3-Chlor-2-hydroxypropyl)-2,4-dicyclohexyl-5-nitroimidazol,
1-(3-Chlor-2-hydroxypropyl)-2,4-dicyclopentyl-5-nitroimidazol,
2,4-Diisopropyl-1-(2,3-dihydroxypropyl)-5-nitroimidazol,
2,4-Bis-(1-methyl-propyl)-1-(2,3-dihydroxypropyl)-5-nitroimidazol,
2,4-Dicyclohexyl-1-(2,3-dihydroxypropyl)-5-nitroimidazol,
2,4-Diisopropyl-1-(4-hydroxybutyl)-5-nitroimidazol,
1-(2-Chlorethyl)-2,4-diisopropyl-5-nitroimidazol,
2,4-Bis-(1-ethyl-propyl)-1-(2-chlorethyl)-5-nitroimidazol,
1-(2-Chlorethyl)-2,4-dicyclopropyl-5-nitroimidazol,
1-(2-Chlorethyl)-2,4-dicyclohexyl-5-nitroimidazol,
2,4-Diisopropyl-1-(2-mercaptoethyl)-5-nitroimidazol,
2,4-Bis-(1-ethyl-propyl)-1-(2-mercaptoethyl)-5-nitroimidazol,
2,4-Dicyclohexyl-1-(2-mercaptoethyl)-5-nitroimidazol,
2,4-Diisopropyl-1-(2-mercaptopropyl)-5-nitroimidazol,
2,4-Diisopropyl-1-(3-mercaptopropyl)-5-nitroimidazol,
2,4-Diisopropyl-1-(bromethyl)-5-nitroimidazol,
2,4-Diisopropyl-1-methoxy-methyl-5-nitroimidazol,
2,4-Diisopropyl-1-methylthiomethyl-5-nitroimidazol,
2,4-Diisopropyl-1-(2-methylthioethyl)-5-nitroimidazol,
3-(2,4-Diisopropyl-5-nitroimidazol-1-yl)-propan-1-sulfonsäure,
3-(2,4-Dicyclohexyl-5-nitroimidazol-1-yl)-propan-1-sulfonsäure,
2-(2,4-Diisopropyl-5-nitroimidazol-1-yl)-ethan-1-sulfonsäure,
1-(2-Aminoethyl)-2,4-diisopropyl-5-nitroimidazol,
1-(2-Aminoethyl)-2,4-bis-(1-ethyl-propyl)-5-nitroimidazol,
1-(2-Diethylaminoethyl)-2,4-diisopropyl-5-nitroimidazol.

Die erfindungsgemäßen Verbindungen ($R^2 \neq H$) zeigen interessante pharmakologische Eigenschaften, neben Wirksamkeit gegenüber Trichomonaden, insbesondere antidepressive, antiasthmatische und antiatherosklerotische Wirksamkeit bei ausgezeichneter Verträglichkeit.
Gegenstand der Erfindung sind daher auch die Verwendung von Verbindungen der Formel I in Arzneimitteln zur Behandlung von Trichomoniasis, Depression, Asthma und Atherosklerose sowie Verfahren zu ihrer Herstellung.

Die erfindungsgemäßen Verbindungen werden nach an sich bekannten Verfahren hergestellt.
Dabei werden Nitroimidazole der Formel II

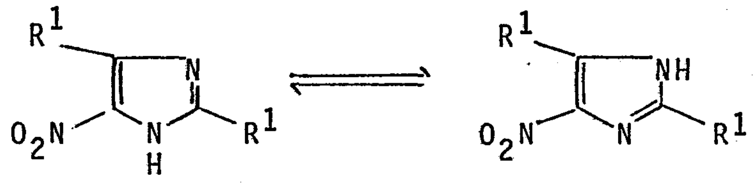

II

in der $R^1$ die in Formel I angegebene Bedeutung hat,

a) mit Verbindungen der Formel $R^2Z$ umgesetzt, wobei $R^2$ die in Formel I angegebenen Bedeutungen hat und Z für Halogen bzw. eine Sulfat- bzw. Tosylatgruppe steht. Die Reaktion erfolgt entweder in alkalischen Medien, wie z.B. Natriumhydrid/Hexamethylphosphorsäuretriamid, Natriumhydrid/Dimethylformamid, Natriumcarbonat/Aceton/Wasser, Kaliumhydroxid/ Aceton/Wasser, Natriummethylat/Ethanol, Kalium-tert.- Butylat/Dimethylsulfoxid oder mittels einer Phasentransfertechnik bzw. in neutralen oder sauren Medien ohne oder mit Lösungsmitteln wie z.B. Toluol, Xylol, Dimethylformamid, Dimethylsulfoxid, Essigsäure, Phosphorsäure, ohne oder mit Katalysatoren, wie z.B. $AlCl_3$, $ZnCl_3$, $BF_3 \cdot Et_2O$, $BCl_3$ usw., bei Temperaturen von 0 - 180 °C,

b) mit Epoxiden mit oder ohne Lösungsmittel in alkalischen, neutralen oder sauren Medien mit oder ohne Katalysator umgesetzt,

c) mit Alkansultonen in einem Lösungsmittel in neutralen, sauren oder alkalischen Medien mit oder ohne Katalysator umgesetzt,

d) indem man ein nach b) durch Umsetzung mit Epihalogenhydrin erhaltenes Produkt mit wässrigem Alkalihydroxid in die 2,3-Epoxypropylverbindung überführt,

e) indem man ein nach b) erhaltenes Produkt nach allgemein bekannten Methoden in ein Halogenid umwandelt,

f) indem man ein nach e) erhaltenes Produkt nach allgemein bekannten Methoden in ein Mercaptan umwandelt,

g) indem man ein Mercaptan erhalten nach f) nach allgemein bekannten Methoden oxydiert.

Gegenstand der Erfindung sind ebenfalls 2,4(5)-Nitroimidazole der Formel II, die erhalten werden, indem man Imidazole der Formel IV

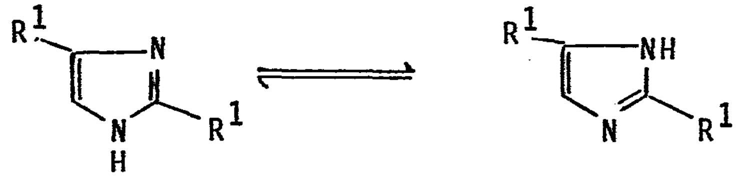

IV

in der $R^1$ die gleiche Bedeutung wie in Formel I hat, nach an sich bekannten Verfahren (R.G. Frager, F.L. Pyman, J. Chem. Soc., London, 115, 217 (1919); G.P. Ellis et al., J. Pharm. Pharmacol. 16, 801 (1964)) in Schwefelsäure/Salpetersäure-Gemischen nitriert.

Als Imidazole der Formel II kommen z.B.:

2,4(5)-Diisopropyl-imidazol
2,4(5)-Di-sec-butyl-imidazol
2,4(5)-Bis-(1-ethyl-propyl)-imidazol
2,4(5)-Bis-(1-propyl-propyl)-imidazol
2,4(5)-Bis-(1-methyl-butyl)-imidazol
2,4(5)-Bis-(1-ethyl-butyl)-imidazol
2,4(5)-Dicyclopropyl-imidazol
2,4(5)-Dicyclopentyl-imidazol
2,4(5)-Dicyclohexyl-imidazol
2,4(5)-Dicycloheptyl-imidazol

in Frage.

Die Imidazole der Formel II sind zum Teil bekannte Verbindungen, die durch Umsetzung von Aldehyden der allgemeinen Formel $R^1$-CHO, in der $R^1$ die in Formel I angegebene Bedeutung besitzt, mit Ammoniak und Blausäure nach an sich bekannten Verfahren (DE-OS 23 15 935) erhalten werden können.

0126183

Die vorliegende Erfindung betrifft ebenfalls pharmazeutische Präparate, welche Verbindungen der Formel I enthalten. Bei den erfindungsgemäßen pharmazeutischen Präparaten handelt es sich um solche zur enteralen wie oralen oder rektalen sowie parenteralen Verabreichung, welche die pharmazeutischen Wirkstoffe allein oder zusammen mit einem üblichen, pharmazeutisch anwendbaren Trägermaterial enthalten. Vorteilhafterweise liegt die pharmazeutische Zubereitung des Wirkstoffes in Form von Einzeldosen vor, die auf die gewünschte Verabreichung abgestimmt sind, wie z.B. Tabletten, Dragées, Kapseln, Suppositorien, Granulate, Lösungen, Emulsionen oder Suspensionen. Die Dosierung der Verbindungen liegt üblicherweise zwischen 1 - 1000 mg pro Dosis, vorzugsweise zwischen 10 - 500 mg je Dosis, und kann ein- oder mehrmals, bevorzugt zwei- bis dreimal täglich, verabreicht werden.

Die Herstellung der erfindungsgemäßen Verbindungen wird durch die folgenden Beispiele näher erläutert.

Beispiel 1

2,4(5)-Diisopropyl-5(4)-nitroimidazol

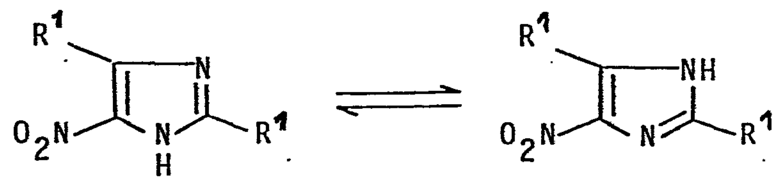

R1 = isopropyl

380 ml 98%ige Schwefelsäure werden in einem Dreihalskolben bis auf ca. - 10 °C abgekühlt.
152 g 2,4(5)-Diisopropyl-imidazol werden unter Rühren dazugegeben. Dann werden langsam 152 ml 100%ige Salpetersäure zugetropft, wobei die Temperatur unter 30 °C gehalten wird. Man rührt noch ca. 2 Stunden bei 45°C. Nach Abkühlen wird die 4fache Menge Eis zugegeben und mit 46%iger Natronlauge neutralisiert. Der pH-Wert wird auf etwa 7,5 - 8 eingestellt. Man extrahiert ca. 5mal mit Dichlormethan, trocknet die vereinigten Dichlormethan-phasen über Natriumsulfat, dampft ein und kristallisiert den Rückstand aus Essigester um.
Ausbeute: 160 g (81,2 %), Fp. 169 - 171 °C.

Analog Beispiel 1 werden folgende 2,4(5)-disubstituierte 5(4)-Nitro-imidazole hergestellt:

2,4(5)-Bis-(1-methyl-propyl)-5(4)-nitroimidazol
Ausbeute 65 %, Fp. 99 - 101 °C.

2,4(5)-Bis-(1-ethyl-propyl)-5(4)-nitroimidazol
Ausbeute 75 %, Fp. 98 - 100 °C.

2,4(5)-Dicyclopropyl-5(4)-nitroimidazol
Ausbeute 82 %, Fp. 178 - 180 °C.

2,4(5)-Dicyclopentyl-5(4)-nitroimidazol
Ausbeute 86 %, Fp. 189 - 192 °C.

2,4(5)-Dicyclohexyl-5(4)-nitroimidazol
Ausbeute 79 %, Fp. 209 - 212 °C

Die als Ausgangsverbindungen eingesetzten 2,4(5)-disubstituierten Imidazole der Formel IV können wie folgt hergestellt werden:

2 Mol des entsprechenden Aldehyds und 200 ml Benzol werden in einem Dreihalskolben mit einem kräftigen Ammoniakstrom begast, wobei die Mischung gerührt und ca. 1 - 2 Stunden unter Abtrennung des gebildeten Wassers am Rückfluß erhitzt wird. Nach Beendigung der Reaktion wird das Benzol unter Normaldruck abgetrennt und der Rückstand entweder fraktioniert destilliert oder sofort weiter eingesetzt. Das Produkt wird in 100 ml trockenem Diethylether gelöst. Eine equimolare Menge HCN wird unter Rühren bei 5 - 15 °C zugetropft und anschließend noch ca. 1 Stunde bei 10 °C gerührt. Dann werden die überschüssige HCN und Ether abgetrennt und das Produkt destilliert. Das Destillat wird mit ca. 500 ml Diethylether verdünnt und die Lösung unter Rühren und Kühlen auf etwa 10 °C mit trockenem HCl gesättigt und anschließend ca. 5 - 6 Stunden stehengelassen. Die Mischung wird mit verdünnter Natronlauge neutralisiert und die organische Phase mit Chloroform 4- bis 5mal extrahiert. Die gesammelten Extrakte werden über $K_2CO_3$ getrocknet, filtriert und das Chloroform abgezogen. Der Rückstand wird umkristallisiert.

Nach diesem Verfahren werden z.B. die folgenden Imidazole hergestellt:

2,4(5)-Diisopropylimidazol
Ausbeute 82 %, Fp. 146 - 147 °C

2,4(5)-Bis-(1-methyl-propyl)-imidazol
Ausbeute 43 %, Fp. 155 - 157 °C

2,4(5)-Bis-(1-ethyl-propyl)-imidazol
Ausbeute 84 %, Fp. 128 - 130 °C

2,4(5)-Dicyclopropylimidazol
Ausbeute 64 %, Fp. 152 - 155 °C

2,4(5)-Dicyclopentylimidazol
Ausbeute 52 %, Fp. 159 - 161 °C

2,4(5)-Dicyclohexylimidazol
Ausbeute 81 %, Fp. 163 - 164 °C

<u>Beispiel 2</u>

<u>2,4-Diisopropyl-1-methyl-5-nitroimidazol</u>

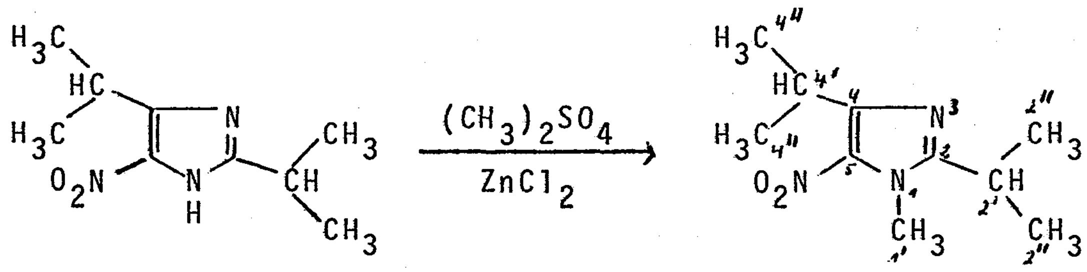


15 g (76,14 mMol) 2,4(5)-Diisopropyl-5(4)-nitroimidazol, 30 ml Dimethylsulfat und 1 g Zinkchlorid werden 5 Minuten unter Rückfluß erhitzt. Nach dem Abkühlen verdünnt man mit Methanol auf das doppelte Volumen und stellt den pH-Wert mittels wässriger Natronlauge auf ca. 6 - 7 ein. Die Lösungsmittel werden eingedampft und der Rückstand in Essigester/Wasser aufgenommen. Nach mehrmaliger Extraktion mit Essigester werden die gesammelten organischen Fraktionen getrocknet und eingedampft. Das Rohprodukt wird über eine mit $Al_2O_3$ 90 Neutral gefüllte Säule eluiert (Toluol).

Ausbeute: 12,5 g eines gelblichen Öls (77 %).

$^1H$ NMR ($CDCl_3$): δ 1,27 ($6H,H_{2''}$), δ 1,33 ($6H,H_{4''}$), δ 3,01 ($1H,H_{2'}$), δ 3,60 ($1H,H_{4'}$), δ 3,85 ($3H,NCH_3$),

$^{13}C$ NMR ($CDCl_3$): δ 20,14 ($C_{4''}$), δ 20,47 ($C_{2''}$), δ 26,21 ($C_{4'}$) δ 27,35 ($C_{2'}$), δ 32,45 ($NCH_3$), δ 134 ($C_5$), δ 152,56 ($C_4$), δ 155,32 ($C_2$)

Beispiel 3

2,5-Diisopropyl-1-methyl-4-nitroimidazol

14 g (71,07 mMol) Nitroimidazol, 50 ml Methanol und 28 ml einer 30 %igen Natriummethylatlösung und 3,4 ml (36 mMol) Dimethylsulfat werden 5 Minuten bei Raumtemperatur gerührt und anschließend 24 Stunden unter Rückfluß erhitzt. Nach dem Eindampfen der Lösungsmittel wird der Rückstand in Chloroform aufgenommen. Die unlöslichen Salze werden abfiltriert und die Lösung erneut eingedampft. Die Reinigung erfolgt über eine $SiO_2$-Säule mittels Dichlormethan. Man erhält 6,3 g 2,4-Diisopropyl-1-methyl-5-nitroimidazol (42 % der Theorie) und 4,0 g 2,5-Diisopropyl-1-methyl-4-nitroimidazol (26,7 % der Theorie). Der Schmelzpunkt des 4-Nitroisomeren liegt bei 78 - 79 °C.

$^1H$ NMR ($CDCl_3$): δ 1,37 ($6H,H_{2''}$), δ 1,42 ($6H,H_{5''}$), δ 3,02 ($1H,H_{2'}$), δ 3,64 ($3H,NCH_3$), δ 3,80 ($1H,H_{5'}$)

$^{13}C$ NMR ($CDCl_3$): δ 18,84 und 20,52 ($C_{2''}$ und $C_{5''}$), δ 24,50 und 25,98 ($C_{2'}$ und $C_{5'}$), S 31,31 ($NCH_3$), δ 138,50 ($C_5$), δ 142,88 ($C_4$), δ 150,90 ($C_2$)

Beispiel 4

2,4-Diisopropyl-1-ethyl-5-nitroimidazol

wird analog Beispiel 1 hergestellt.
Gelbliches Öl, Ausbeute 65 %.

$^1H$ NMR ($CDCl_3$): δ 1,28 ($6H,H_{2''}$), δ 1,36 ($6H,H_{4''}$), δ 1,40 ($3H,H_{1''}$), δ 3,63 ($1H,H_{4'}$), δ 4,33 ($2H,NCH_2$)

$^{13}C$ NMR ($CDCl_3$): δ 15,94 ($C_{1''}$), δ 20,79 ($C_{2''}$), δ 21,20 ($C_{4''}$), δ 26,39 ($C_{2'}$), δ 27,73 ($C_{4'}$), δ 40,68 ($NCH_2$), δ 133,31 ($C_5$), δ 154,00 ($C_4$), δ 155,55 ($C_2$)

## Beispiel 5

**2,4-Diisopropyl-1-(2-hydroxyethyl)-5-nitroimidazol**

Methode A

Zu 2 g (10,1 mMol) 2,4(5)-Diisopropyl-5(4)-nitroimidazol in 100 ml Dichlormethan werden 1,5 g (10,5 mMol) wasserfreies Aluminiumchlorid zugegeben. Bei 0 °C werden dann 5 ml Ethylenoxid zugetropft. Nach 3stündigem Rühren bei 0 °C wird das Reaktionsgemisch über Nacht bei Raumtemperatur stehengelassen. Das Gemisch wird mit Eiswasser hydrolysiert und die wässrige Phase auf ca. pH 6 - 7 gebracht. Nach Trennung der Phasen wird die Wasserphase nochmals mit 100 ml Dichlormethan extrahiert. Die vereinigten organischen Phasen werden über Natriumsulfat getrocknet und eingedampft. Das zurückbleibende Öl wird mit 10 ml trockenem Ether ausgerührt und das dabei ausfallende Ausgangsimidazol abfiltriert. Der Ether wird eingedampft. Ausbeute 2,2 g ( $>$ 90 %).

Methode B

2 g (10,1 mMol) 2,4(5)-Diisopropyl-5(4)-nitroimidazol werden in 10 ml Eisessig und 3 ml Chloroform gelöst. 1,25 ml (1,44 g) $BF_3 \cdot Et_2O$ werden zugetropft und die Mischung auf 0 - 5 °C gekühlt. Nach dem Zutropfen von 5 ml Ethylenoxid wird eine Stunde bei 0 °C und anschließend bei Raumtemperatur über Nacht weitergerührt. Die Lösungsmittel werden abdestilliert und der Rückstand in 1 N Natronlauge aufgenommen. Nach Einstellung des pH auf 6 - 7 wird mit Dichlormethan extrahiert. Die organische Phase wird getrocknet und eingedampft. Reinigung des Endproduktes wie bei Methode A.

Ausbeute $>$90 %.

$^1H$ NMR ($CDCl_3$): δ 1,29 (6H,$H_{2''}$), δ 1,33 (6H,$H_{4''}$), δ 2,1 (1H, OH), δ 3,18 (1H,$H_{2'}$), δ 3,63 (1H,$H_{4'}$), δ 3,97 (2H,$CH_2OH$), δ 4,48 (2H,$NCH_2$)

$^{13}C$ NMR ($CDCl_3$): δ 20,22 ($C_{2''}$), δ 20,51 ($C_{4''}$), δ 25,88 ($C_{4'}$), δ 27,31 ($C_{2'}$), δ 47,16 ($NCH_2$), δ 60,31 ($CH_2OH$) δ 133,12 ($C_5$), δ 153,70 ($C_4$), δ 157,12 ($C_2$)

## Beispiel 6

**2,4-Bis-(1-ethyl-propyl)-1-(2-hydroxyethyl)-5-nitroimidazol**

Hergestellt analog Beispiel 4, Methode A aus 2,4(5)-Bis-(1-ethyl-propyl)-5(4)-nitroimidazol und Ethylenoxid.

Gelbliches Öl, Ausbeute 86 %.

$^1$H NMR ($CDCl_3$): δ 0,80 und 0,83 (12H,$CH_3$), δ 1,6 - 1,9 (8H, $CH_2$-$CH_3$), δ 2,15 (1H,OH), δ 2,78 (1H,$H_{2'}$), ~~δ 3~~,38 (1H,$H_{4'}$), δ 3,94 (2H,$CH_2O$), δ 4,48 (2H,$NCH_2$)

## Beispiel 7

**2,4-Dicyclohexyl-1-(2-hydroxyethyl)-5-nitroimidazol**

Hergestellt analog Beispiel 4, Methode A aus 2,4(5)-Dicyclohexyl-5(4)-nitroimidazol und Ethylenoxid.

Öl, Ausbeute 81 %

$^1$H NMR ($CDCl_3$): δ 1,5 - 2 (20H,$CH_2$), δ 2,20 (1H,OH), δ 2,76 (1H,$H_{2'}$), δ 3,40 (1H,$H_{4'}$), δ 3,91 (2H,$CH_2O$), δ 4,51 (2H,$NCH_2$)

## Beispiel 8

**2,4-Diisopropyl-1-(2-hydroxypropyl)-5-nitroimidazol**

Hergestellt analog Beispiel 4, Methode A aus 2,4(5)-Diisopropyl-5(4)-nitroimidazol und 1,2-Epoxypropan.

Ausbeute 89 %, Fp. 85 - 90 °C

$^1$H NMR ($CDCl_3$): δ 1,30 (6H,$H_{2''}$), δ 1,31 (3H,$CH_3$), δ 1,36 (6H,$H_{4''}$), δ 2,1 (1H,OH), δ 3,18 (1H,$H_{2'}$), δ 3,50 (1H,<u>CH</u>OH), δ 3,62 (1H,$H_{4'}$), δ 4,45 (2H,$NCH_2$)

## Beispiel 9

### 1-(3-Chlor-2-hydroxpropyl)-2,4-diisopropyl-5-nitroimidazol

10 g (50,76 mMol) 2,4(5)-Diisopropyl-5(4)-nitroimidazol und 250 mg des Natriumsalzes derselben Verbindung in 50 ml Epichlorhydrin werden 30 Minuten unter Rückfluß erhitzt. Das überschüssige Epichlorhydrin wird im Vakuum abdestilliert. Die Reinigung des Rückstandes erfolgt durch Säulenchromatographie und Umkristallisation (Hexan-Toluol).

Ausbeute 9,5 g (63,2 %), Fp. 121 - 123 °C

$^1$H NMR ($CDCl_3$): δ 1,27 ($6H,H_{2''}$), δ 1,36 ($6H,H_{4''}$), δ 2,82 (1H,OH), δ 3,17 ($1H,H_{2'}$), δ 3,61 ($1H,H_{4'}$), δ 3,71 ($2H,CH_2Cl$), δ 4,25 (1H,<u>CH</u>OH), δ 4,30 und 4,57 ($2H,NCH_2$)

## Beispiel 10

### 2,4-Diisopropyl-1-(2,3-epoxypropyl)-5-nitroimidazol

5 g (17,27 mMol) 1-(3-Chlor-2-hydroxypropyl)-2,4-diisopropyl-5-nitroimidazol werden mit 50 ml 5 %iger Natronlauge versetzt. Die Mischung wird 15 Minuten auf 70 - 80 °C erhitzt, dann abgekühlt und neutralisiert. Das Produkt wird mittels Chloroform extrahiert. Nach der üblichen Aufarbeitung erhält man 4,3 g eines gelblichen Öls, Ausbeute 98,4 %.

$^1$H NMR ($CDCl_3$): δ 1,25 - 1,37 ($12H,CH_3$), δ 2,55 und 2,88 (1H+1H,$\underline{CH_2}$-CH), δ 3,04 ($1H,H_{2'}$), δ 3,37 (1H,<u>CH</u>-$CH_2$), δ 3,62 ($1H,H_{4'}$), δ 4,14 und 4,83 ($1H+1H,NCH_2$)

$^{13}$C NMR ($CDCl_3$): δ 20,87 und 20,94 ($CH_3$), δ 26,49 ($C_{4'}$), δ 27,96 ($C_{2'}$), δ 45,36 ($NCH_2$), δ 47,15 (—$\underline{CH_2}$), δ 50,96 (<u>CH</u>-$CH_2$), δ 133,21 ($C_5$), δ 154,34 ($C_4$), δ 157,21 ($C_2$)

Beispiel 11

1-(2-Chlorethyl)-2,4-diisopropyl-5-nitroimidazol

Zu 10 g (50,76 mMol) 2,4-Diisopropyl-1-(2-hydroxyethyl)-5-nitroimidazol werden unter Kühlung bei 0 °C 10 ml Thionylchlorid zugetropft. Das Reaktionsgemisch wird 4 Stunden weitergerührt. Nach Zugabe von 100 ml Toluol wird das Lösungsmittel im Vakuum abdestilliert und der Rückstand über eine Kieselgelsäule mit Chloroform als Elutionsmittel gereinigt. Nach Aufarbeitung verbleiben 5,8 g eines gelblichen Öls, Ausbeute 74 %.

$^1$H NMR ($CDCl_3$): δ 1,27 ($6H,H_{2''}$), δ 1,38 ($6H,H_{4''}$), δ 3,18 ($1H,H_{2'}$), δ 3,60 ($1H,H_{4'}$), δ 3,81 ($2H,CH_2Cl$), δ 4,59 ($2H,NH_2$)

Beispiel 12

2,4-Diisopropyl-1-(2-mercaptoethyl)-5-nitroimidazol

10 g (38,53 mMol) 1-(2-Chlorethyl)-2,4-diisopropyl-5-nitroimidazol und 3,8 g (50 mMol) Thioharnstoff in 100 ml 95%igem Ethanol werden 1 Stunde unter Rückfluß erhitzt. Nach dem Abkühlen werden 4,6 g Natriumhydroxid, gelöst in 50 ml Wasser, zugegeben. Das Reaktionsgemisch wird dann wieder 1 Stunde unter Rückfluß erhitzt und nach Abkühlen mit $CO_2$ neutralisiert. Nach der Extraktion mit Ether und der üblichen Aufarbeitung und Reinigung durch Säulenchromatographie erhält man 7,3 g eines Öls, Ausbeute 73,7 %.

$^1$H NMR ($CDCl_3$): δ 1,28 ($6H,H_{2''}$), δ 1,35 ($6H,H_{4''}$), δ 1,3 (1H,SH), δ 1,13 ($1H,H_{2'}$), δ 3,63 ($1H,H_{4'}$), δ 3,85 ($2H,CH_2S$), δ 4,60 ($2H,NCH_2$)

Beispiel 13

2-(2,4-Diisopropyl-5-nitroimidazol-1-yl)-ethansulfonsäure

Zu 2 g (7,78 mMol) 2,4-Diisopropyl-1-(2-mercaptoethyl)-5-nitroimidazol in 20 ml Wasser werden 7 ml 37%ige Salzsäure zugegeben. Die Temperatur steigt dabei auf ca. 30 °C an. Dann werden 3,73 g (1,19 ml) Brom zugetropft, wobei sich die Temperatur auf 35 °C erhöht. Nach 1 Stunde Rühren bei Raumtemperatur wird das Reaktionsgemisch auf Eiswasser gegossen und mit Chloroform extrahiert. Nach üblicher Aufarbeitung erhält man 2,1 g Substanz, Ausbeute 88 %, Fp. 50 - 52 °C.

$^1$H NMR ($CDCl_3$): $\delta$ 1,25 (6H,$H_{2''}$), $\delta$ 1,36 (6H,$H_{4''}$), $\delta$ 3,12 (1H,$H_{2'}$), $\delta$ 3,62 (1H,$H_{4'}$), $\delta$ 3,81 (2H,$CH_2$-$SO_3$) $\delta$ 4,59 (2H,$NCH_2$)

Beispiel 14

2,4-Diisopropyl-1-methoxymethyl-5-nitroimidazol

Eine Mischung aus 2,5 g (12,5 mMol) 2,4(5)-Diisopropyl-5(4)-nitroimidazol, 4 g (10 mMol) Methyltridecylammoniumchlorid, 1 g (12,7 mMol) Chlormethylmethylether, 10 ml 50%iger NaOH und 80 ml Toluol wird 8 Stunden unter Rückfluß erhitzt. Nach Wasserzugabe wird die organische Phase abgetrennt und mit Wasser nachgewaschen. Nach üblicher Aufarbeitung wird der Rückstand durch Säulenchromatographie gereinigt. Gelbliches Öl, Ausbeute 1,46 g (48,4 %).

$^1$H NMR ($CDCl_3$): $\delta$ 1,25 (6H,$H_{2''}$), $\delta$ 1,35 (6H,$H_{4''}$), $\delta$ 3,10 (1H,$H_{2'}$), $\delta$ 3,38 (3H,$CH_3O$), $\delta$ 3,63 (1H,$H_{4'}$), $\delta$ 5,66 (2H,$NCH_2$)

Beispiel 15

2,4-Diisopropyl-1-methylthiomethyl-5-nitroimidazol

Hergestellt analog Beispiel 12 aus 2,4(5)-Diisopropyl-5(4)-nitroimidazol und Chlormethylmethylsulfid.

Öl, Ausbeute 52,5 %

$^1H$ NMR ($CDCl_3$): δ 1,24 ($6H,H_{2''}$), δ 1,36 ($6H,H_{4''}$), δ 2,17 ($3H,SCH_3$), δ 3,11 ($1H,H_{2'}$), δ 3,63 ($1H,H_{4'}$), δ 4,64 ($2H,NCH_2$)

Beispiel 16

3-(2,4-Diisopropyl-5-nitroimidazol-1-yl)-propansulfonsäure

Zu einer Suspension von 0,66 g (20,2 mMol) Natriumhydrid (80%ig in Öl) in 10 ml Dimethylformamid werden 3,9 g (19,8 mMol) 2,4(5)-Diisopropyl-5(4)-nitroimidazol, gelöst in 10 ml Hexamethylphosphorsäuretriamid, zugetropft. Nach 5 Stunden Rühren bei Raumtemperatur werden 2,44 g (20 mMol) 1,3-Propansulton in 10 ml Hexamethylphosphorsäuretriamid zugetropft und 8 Stunden auf 80 °C erhitzt. Das Reaktionsgemisch wird auf Eiswasser hydrolysiert und mit Chloroform extrahiert. Die Chloroformphase wird 3mal mit 10%iger Natriumcarbonatlösung extrahiert. Die Natriumcarbonatphase wird mit Ether ausgeschüttelt und nach der Abtrennung angesäuert. Nach erneuter Extraktion mit Chloroform und üblicher Aufarbeitung erhält man 1,45 g eines gelblichen Öls, Ausbeute 22,9 %.

$^1H$ NMR ($CDCl_3$): δ 1,26 ($6H,H_{2''}$), δ 1,36 ($6H,H_{4''}$), δ 1,62 ($2H,NCH_2$-$\underline{CH_2}$), δ 3,14 ($1H,H_{2'}$), δ 3,63 ($1H,H_{4'}$) δ 3,80 ($2H,CH_2SO_3$), δ 4,61 ($2H,NCH_2$)

Beispiel 17

1-(2-Aminoethyl)-2,4-diisopropyl-5-nitroimidazol

A 2,4-Diisopropyl-1-(2-phthalimidoethyl)-5-nitroimidazol

Eine Lösung von 5,91 g (30 mMol) 2,4(5)-Diisopropyl-5-nitroimidazol in 15 ml Dimethylformamid wird zu einer Suspension von 1,0 g Natriumhydrid (80%ig in Mineralöl)

in 15 ml Dimethylformamid zugetropft. Die Mischung wird 6 Stunden bei Raumtemperatur gerührt. Anschließend wird 7,62 g (30 mMol) 2-Bromethylphthalimid in 50 ml Hexamethylphosphorsäuretriamid zugetropft. Nach 15 Stunden Rühren bei 60 °C wird das Gemisch abgekühlt und auf Eiswasser geschüttet. Nach mehrmaliger Extraktion mit Chloroform und üblicher Aufarbeitung und Reinigung durch Säulenchromatographie erhält man 5,45 g Substanz, Fp. 100 - 105 °C.

B 1-(2-Aminoethyl)-2,4-diisopropyl-5-nitroimidazol

Das nach A erhaltene Produkt wird mit 50 ml wässriger Bromwasserstofflösung (48%ig) 16 Stunden unter Rückflußtemperatur erhitzt. Nach dem Abkühlen wird die ausgefallene Phthalsäure abfiltriert und das Filtrat eingedampft. Der Rückstand wird 2mal aus Methanol-Diisopropylether umkristallisiert. Das Amin wird aus dem Dihydrobromid mit 10%iger Natriumcarbonatlösung freigesetzt. Nach der üblichen Aufarbeitung erhält man 1,3 g Öl, Ausbeute 18 %.

$^1H$ NMR ($CDCl_3$): δ 1,27 (6H,$H_{2''}$), δ 1,34 (6H,$H_{4''}$), δ ~ 2,5 (2H,$NH_2$), δ 3,19 (1H,$H_{2'}$), δ 3,63 (1H,$H_{4'}$), δ 2,85 (2H,$\underline{CH_2}$-$NH_2$), δ 4,43 (2H,$NCH_2$)

## Beispiel 18

2,4-Diisopropyl-1-(2-dimethylaminoethyl)-5-nitroimidazol

wird analog Beispiel 16 A hergestellt aus 2,4(5)-Diisopropyl-5(4)-nitroimidazol und Chlorethyldimethylamin.

Ausbeute 65,3 %, Öl.

$^1H$ NMR ($CDCl_3$): δ 1,27 (6H,$H_{2''}$), δ 1,36 (6H,$H_{4''}$), δ 2,4 (6H,$N\underline{(CH_3)_2}$), δ 2,63 (2H,$\underline{CH_2}$-$N(CH_3)_2$), δ 3,01 (1H,$H_{2'}$), δ 3,62 (1H,$H_{4'}$), δ 4,42 (2H,N-$CH_2$)

Anmelder: A. Nattermann & Cie GmbH
Nattermannallee 1, 5000 Köln 30

Titel: Substituierte 5(4)-Nitroimidazole sowie Verfahren zu ihrer Herstellung

Patentansprüche

1. Substituierte 5(4)-Nitroimidazole der Formel I

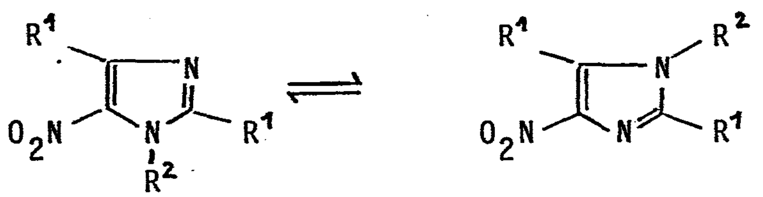

I

worin

$R^1$ die Gruppe $-CHR^3R^4$ oder eine Cycloalkylgruppe mit 3 - 7 Kohlenstoffatomen

$R^3$ einen Alkylrest mit 1 - 18 Kohlenstoffatomen

$R^4$ einen Alkylrest mit 1 - 18 Kohlenstoffatomen

$R^2$ Wasserstoff, einen geradkettigen oder verzweigten, gesättigten oder ungesättigten Alkylrest mit 1 - 16 Kohlenstoffatomen, eine Epoxyalkylgruppe oder die Gruppe -A-Y in der
 |
 Y

A eine geradkettige oder verzweigte Alkylengruppe, die ein- bzw. zweifach substituiert sein kann,

Y ein Wasserstoffatom, $-XR^5$ oder Halogen

X O, S, $SO_2$, $SO_3$ oder $NR^5$

$R^5$ ein Wasserstoffatom oder einen geradkettigen oder verzweigten Alkylrest mit 1 - 8 Kohlenstoffatomen

bedeuten.

2. 1,2,4(5)-Substituierte 5(4)-Nitroimidazole der Formel I nach Anspruch 1, dadurch gekennzeichnet, daß $R^1$ eine $-CHR^3R^4$-Gruppe ist, in der $R^3$, $R^4$ gleich oder verschieden einen Methyl- oder Ethylrest bedeuten.

3. 1,2,4(5)-Substituierte 5(4)-Nitroimidazole der Formel I nach Anspruch 1, dadurch gekennzeichnet, daß $R^1$ eine Cycloalkylgruppe mit 3 - 7 Kohlenstoffatomen bedeutet.

4. 1,2,4(5)-Substituierte 5(4)-Nitroimidazole der Formel I gemäß den Ansprüchen 1,2 oder 3, dadurch gekennzeichnet, daß $R^2$ eine $C_{1-4}$-Alkylgruppe, eine 2,3-Epoxypropylgruppe oder die Gruppe $-\underset{|}{\overset{}{A}}-Y$, in der
Y

A entweder eine geradkettige oder verzweigte monosubstituierte Alkylengruppe (ein Y = H)

$$-(CH_2)_n-Y, \quad -\overset{CH_3}{\overset{|}{CH}}-(CH_2)_m-Y, \quad -CH_2-\overset{CH_3}{\overset{|}{CH}}-(CH_2)_m-Y$$

oder eine disubstituierte Alkylengruppe wie

$$-CH_2-\underset{Y}{\underset{|}{CH}}-CH_2-Y$$

Y Wasserstoff, $-XR^5$ oder Halogen
X O, S, $SO_2$, $SO_3$ oder $-NR^5$
$R^5$ Wasserstoff oder einen geradkettigen oder verzweigten Alkylrest mit 1 - 8 Kohlenstoffatomen
m 0 - 3 und
n 1 - 4

bedeuten.

5. 1,2,4(5)-Substituierte 5(4)-Nitroimidazole der Formel I nach einem der Ansprüche 1, 2 oder 4, dadurch gekennzeichnet, daß

$R^1$ Isopropyl

$R^2$ Methyl, Ethyl, Propyl, Epoxypropyl oder die Gruppe -A-Y in der
|
Y

A $-(CH_2)_n-$, $-\overset{CH_3}{CH}-(CH_2)_m-$, $-CH_2-\overset{CH_3}{CH}-(CH_2)_m-$, $-CH_2-\overset{|}{CH}-CH_2-$

Y ein Wasserstoffatom, $XR^5$ oder Halogen

X O, S, $SO_2$, $SO_3$ oder $NR^5$

n 1-3 und

m 0 oder 1

$R^5$ wie in Anspruch 1 oder 4, bedeuten.

6. Verfahren zur Herstellung von 1,2,4(5)-substituierten 5(4)-Nitroimidazolen der Formel I nach einem oder mehreren der Ansprüche 1 - 5, dadurch gekennzeichnet, daß Nitroimidazole der Formel II

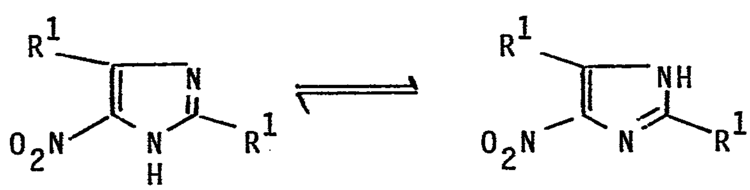

II

in der $R^1$ die in Formel I angegebene Bedeutung hat

a) mit Alkylierungsmitteln der Formel III umsetzt

$R^2$-Z III

wobei $R^2$ die in Formel I angegebenen Bedeutungen hat und Z ein Halogen oder einen Sulfat- bzw. Tosylatrest darstellt,

b) mit Epoxiden mit oder ohne Lösungsmittel in alkalischen, neutralen oder sauren Medien mit oder ohne Katalysator umsetzt,

c) mit Alkansultonen in einem Lösungsmittel in neutralen, sauren oder alkalischen Medien mit oder ohne Katalysator umsetzt,

d) indem man ein nach b) durch Umsetzung mit Epihalogenhydrin erhaltenes Produkt mit wässrigem Alkalihydroxid in die 2,3-Epoxipropylverbindung überführt,

e) indem man ein nach b) erhaltenes Produkt nach allgemein bekannten Methoden in ein Halogenid umwandelt,

f) indem man ein Produkt erhalten nach e) nach allgemein bekannten Methoden in ein Mercaptan umwandelt,

g) indem man ein Mercaptan erhalten nach f) nach allgemein bekannten Methoden oxydiert.

7. Verfahren zur Herstellung von 2,4(5)-substituierten 5(4-)-Nitroimidazolen der Formel I mit $R^2$ = H, dadurch gekennzeichnet, daß man Imidazole der Formel IV

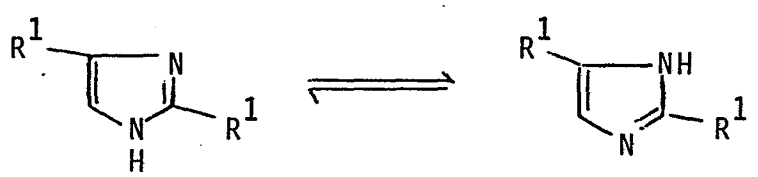

IV

nach an sich bekannten Verfahren mit Salpetersäure nitriert.

8. Pharmazeutische Präparate, dadurch gekennzeichnet, daß sie eine Verbindung der Formel I gemäß den Ansprüchen 1 - 5 als Wirkstoff im Gemisch mit üblichen pharmazeutischen Hilfs- und Trägerstoffen enthalten.

Europäisches Patentamt

# EUROPÄISCHER RECHERCHENBERICHT

0126183

Nummer der Anmeldung

EP 83 11 2433

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | DE-A-2 414 280 (BASF) * Seiten 1-3 * | 1,3,6 | C 07 D 233/92<br>C 07 D 233/93<br>C 07 D 233/94<br>C 07 D 233/95<br>C 07 D 405/06<br>A 61 K 31/415 |
| | --- | | |
| X | FR-A-2 367 066 (BASF) * Seite 2, Zeile 17 - Seite 4, Zeile 30 * | 1,2,7 | |
| | ----- | | |
| | | | RECHERCHIERTE SACHGEBIETE (Int. Cl. 3) |
| | | | C 07 D 233/00<br>C 07 D 405/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 03-09-1984 | DE BUYSER I.A.F. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82